Europäisches Patentamt

European Patent Office

Office européen des brevets

(11) Publication number: **0 296 252**
**A1**

# EUROPEAN PATENT APPLICATION

published in accordance with Art. 158(3) EPC

(21) Application number: **88900791.0**

(22) Date of filing: **08.01.88**

Data of the international application taken as a basis:

(86) International application number:
**PCT/JP88/00012**

(87) International publication number:
**WO88/05152 (14.07.88 88/15)**

(51) Int. Cl.³: **G 01 B 11/24**
**G 01 B 9/08**

(30) Priority: **08.01.87 JP 2471/87**

(43) Date of publication of application:
**28.12.88 Bulletin 88/52**

(84) Designated Contracting States:
**DE FR GB**

(71) Applicant: **MITOH CO., LTD.**
**256, Tomitakeshinden Ryuo-cho**
**Nakakoma-gun Yamanashi 400-01(JP)**

(71) Applicant: **YS-DENSHI INDUSTRY CO., LTD.**
**19-18, Kokubo 5-chome**
**Kofu-shi Yamanashi 400(JP)**

(72) Inventor: **SUGITA, Yoshio**
**19-18, Kokubo 5-chome**
**Kofu-shi Yamanashi 400(JP)**

(74) Representative: **Patentanwaltsbüro Cohausz & Florack**
**Schumannstrasse 97**
**D-4000 Düsseldorf 1(DE)**

(54) **OPTICAL MEASURING INSTRUMENT.**

(57) An optical measuring instrument wherein a turn-table for mounting a work is provided on a moving stage to automatically measure the contour and shape of the work, and the moving stage and the turn-table are controlled while being interlocked to the operation of an edge sensor for measuring the contour.

EP 0 296 252 A1

- 1 -

## SPECIFICATION

TITLE OF THE INVENTION

Optical Measuring Device

TECHNICAL FIELD

This invention relates to an optical measuring device for performing an automatic measurement of a contour, a shape, a degree of parallelization or the like, of a workpiece.

BACKGROUND ART

As already known, both a contact method and a non-contact method are conventionally generally used to measure a size of a workpiece, and the optical measuring device of this invention mainly adopts the non-contact method utilizing a method of a transformation of coordinates system.

But, in the non-contact method, it is very difficult to coincide the reference line of the measurement device and the reference line of the workpiece being measured in the non-contact operation, but to obtain a precise measurement, both reference lines must coincide with each other as much as possible. Accordingly, the operation of coinciding both reference lines with each other must be accurate.

Further, in this method, the device should have a superior repeatability, to avoid variations of data of the size of the workpiece to be measured, which can occur each time the measurement operation is carried out.

Heretofore, as this kind of optical measuring device, a device having a projector, an edge sensor provided with a transducer enabling data obtained to be displayed in a digital form, a controller provided with a CPU therein, and a data processing device, is generally known.

In this prior art, the projector has a rotational screen, a projecting lens, an optical system provided with a light source, and a table for moving the workpiece mounted thereon in both the X-axis and Y-axis directions.

Accordingly, usually a workpiece is mounted on the table in parallel with the X-axis and Y-axis (the reference line of the workpiece is made parallel to the X-axis or the Y-axis) and then the measurement operation for measuring a contour, a shape, a degree of parallelization or the like of a workpiece is carried out. But, heretofore, this operation for obtaining a parallelism of the workpiece is performed by applying a calculation of an apparent image.

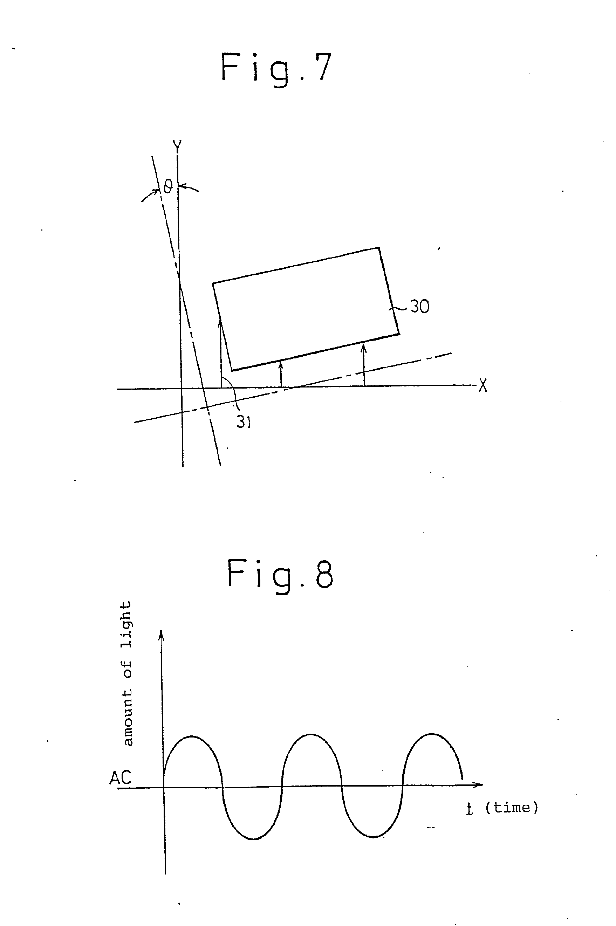

Namely, in an initial stage, the workpiece mounted on a table is usually positioned with certain incli-nation having an angle $\theta$ to the X-axis or Y-axis as shown in Figure 7, but since the conventional table can be driven only in a direction parallel to the X-axis and the Y-axis, the measuring of a contour, a shape, a degree of parallelization or the like of a workpiece 30 is carried out by drawing an imaginary X-axis and Y-axis for the workpiece 30, using a calculation obtained by a computer in a data processing device, (for example, the data measured is multiplied by tan $\theta$).

But, as described above, since the table is driven only in the direction parallel to the X-axis and Y-axis, an actual scanning of the workpiece 30 by the optical system and the sensor will be carried out along a direction indicated by an arrow 31 in Figure 7.

At this time, if the workpiece has a rectangular shape, only a slight error would occur at a long side of the rectangular shape of the workpiece, but at a short side of the rectangular shape of the workpiece 30, a condition arises in which many errors easily occur because the short side thereof has a greater inclination to the scanning direction.

This situation is completely outside the conception that, when performing the measuring of a workpiece on

0296252

the order of microns, it is preferable only to increase the precision of the sensor used in this operation.

Further, another measuring method in which, after the inclined angle θ has been measured, a size of each respective portion of the workpiece is measured by coinciding the reference line of the workpiece to the X-axis or the Y-axis by manually rotating the workpiece to match the calculated data, is also used.

But in this measuring method depending upon a manual operation, the dispersion of data obtained will be large, and therefore, accurate measurement data cannot be obtained, and further, a lot of time is required for the measurement.

The object of the invention is to provide an optical measuring device by which all of the drawbacks mentioned above are eliminated and in which the operation of making the reference line of the workpiece parallel to the X-axis or Y-axis can be carried out extremely rapidly and accurately without the need for a manual operation and the precision thereof is unchanged even during a cyclic operation, and further, data of the dimensions of the workpiece can be simultaneously measured.

DISCLOSURE OF THE INVENTION

To attain the object of this invention, the optical measuring device according to this invention is basically characterized in that the optical measuring device comprises a movable stage, a rotatable table on which a workpiece is mounted and provided on the stage, a optical system for illuminating the workpiece with a light, an edge sensor for reading a contour of the workpiece configurated by the optical system, and a data processor which carries out at least one controlling operation selected from an operation for controlling a movement of the stage corresponding to information from the edge sensor, an operation for controlling a rotational movement of the rotatable table, and an operation for displaying the data obtained from measuring the workpiece.

Moreover, in this invention, a circuit for compensating a variance of a amount of light from the light source of the optical system is further provided, and thus if the amount of light has been changed for any reason, such a change has no affect on the measurement data, and accordingly, accurate data can be always obtained.

BRIEF DESCRIPTION OF THE DRAWINGS

Figure 1 is a perspective view of one example of the optical measuring device of this invention.

Figure 2 is a partially enlarged cross sectional view indicating a relationship between a movable stage and a rotatable table.

Figure 3 is a schematic diagram of a discriminating circuit including an amount of light compensating circuit.

Figure 4 is a plane view of a scanning operation for parallelizing the workpiece.

Figure 5 shows a coordinate system for the operation in which the workpiece is parallelized.

Figure 6 is a schematic view of an example of an output of measurement results.

Figure 7 shows a positional relationship between the workpiece and the coordinate in the prior art.

Figure 8 and Figure 9 are graphs indicating a variance of an amount light of a light source in the prior art in this invention, respectively.

Figure 10 to Figure 15 showing examples of the applications of the measurement of a workpiece utilizing a device of this invention.

BEST MODE OF CARRYING OUT THE INVENTION

Hereafter, the optical measuring device of this invention will be explained with reference to the accompanying drawings.

In this invention, the operation of making the reference line of a workpiece parallel with the X-axis or Y-axis and the operation for measuring a shape and size of a workpiece can be carried out extremely rapidly and accurately by combining a movable stage and a rotatable table, and further, by controlling both thereof with a controlling means utilizing a computer or the like, compared with a conventional optical measuring device having only a movable stage.

In this invention, a workpiece to be measured is mounted and fixed on the rotatable table.

Further, in this invention, an operation for measuring the position of each contour portion of a work by tracing a shape of the contour by a shadow formed by a light obtained from a light source, by an edge sensor is carried out, and a calculating operation is carried out to determine the angle θ between a reference line of the workpiece and the X-axis or the Y-axis, after the measurement data is input to a calculator, for example, a computer, and the data processed therein.

Thereafter, the operation for making the reference line of a workpiece parallel with the X-axis or Y-axis is carried out by controlling the rotatable table to rotate by a certain angle corresponding to the results of the calculation.

An example of this invention will be described hereunder.

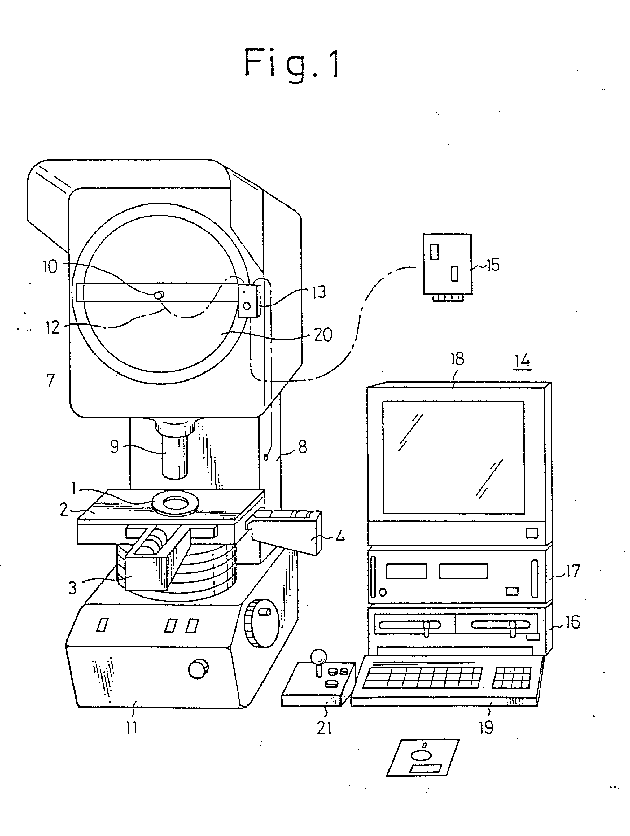

In Figure 1, a rotatable table 1 for mounting a workpiece thereon and which can move in three dimensions is provided, and this rotatable table 1 is mounted on a movable stage 2 provided on a base portion 11 of the device.

The movable stage 2 is controlled to be moved in both the X-axis and Y-axis directions by a stepping motor 3 which moves the stage in the X-axis direction, and a stepping motor 4 which moves the stage in the Y-axis direction.

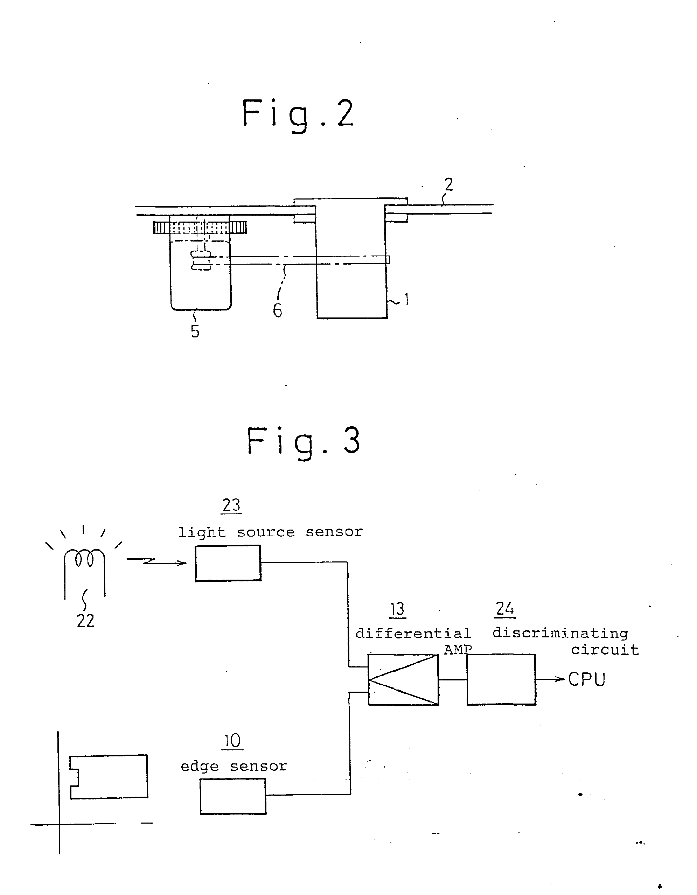

Further, the rotation of the rotatable table 1 mounted on the movable stage 2 is controlled by a stepping motor 5 mounted on the movable stage 2 through a timing belt 6 as shown in Figure 2.

Each of the stepping motors 3, 4 and 5 is controlled by a suitable program memorized in a computer, as explained later, respectively.

Further, in an optical system of this invention, a projecting lens 9 having a zoom function, a light source 22 (not shown) arranged under the rotatable table 1 and illuminating the workpiece mounted on the rotatable table, and a reflecting mirror (not shown) reflecting the light emitted from the light source 22 for illuminating the workpiece, through the projecting lens 9 having the zoom function, onto a projecting screen 20 are mainly provided.

A projection device 7 having the mirror and the projecting lens 9 provided over the rotatable table 1 is provided on a supporting portion 8 mounted on the base portion 11 of the device.

On a center portion of the projecting screen 20 of the projection device 7, an edge sensor 10 for detecting a variation of a value of the light is provided, which edge sensor 10 can read an inclination and a contour shape of a workpiece illuminated by the optical system.

The edge sensor 10 is connected to an differential amplifier 13 through a optical fiber 12 attached to the back end thereof, as shown in Figure 3, and further, a light source sensor 23 for detecting an amount of light from the light source 22 consisting of, for example, a halogen lamp, is also connected to the differential amplifier 13 as shown in Figure 3, so that in this system, not only the measurement of the light value on the projecting screen 20 by the edge sensor 10 but also the compensation of a change of the amount of light from the light source 22 by the light source sensor 23 are carried out.

Namely, to prevent the amount of light to be

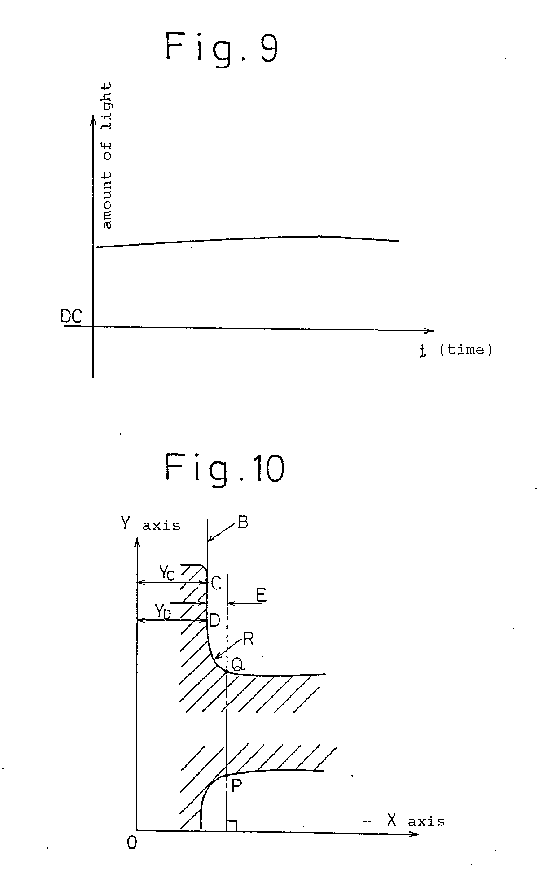

changed in its intensity caused by the alternating current source as shown in Figure 8, such alternating current is converted into a direct current by the AC-DC convertor as shown in Figure 9 but it is necessary for the variation of the amount of the light to be compensated by the light source sensor 23 because it is unavoidable for the amount of the light to be changed caused by a variation of the halogen lamp per se depending upon the variationof its temperature condition based upon the time elapsing.

A method of adjusting the amount of light is carried out in such a way that the data output from the edge sensor 10 is compensated by the data output from the light source sensor 23, utilizing the amplifier 13, and the compensated data is, for example, transferred to an edge discriminating circuit 24 to discriminate the edge, as shown in Figure 3.

Further, the method of adjusting the amount of light may be carried out in such a way that a variation of the amount of light from the light source 22 is read at a predetermined time interval and the amount of light is compensated by adjusting a voltage applied to the light source, by using a stabilized power source separately provided, corresponding to the resultant data.

The measurement data representing an inclination and a shape of the workpiece consisting of electrical signals read by the edge discriminating circuit 24 and the amount of light adjusted by the differential amplifier 13, is then processed in the control and data processing portion 14.

The control and data processing portion 14 is comprised of an input/output card 15 (referred to hereafter as I/O card) for reading the data described above, a main frame 16 including a computer (referred to hereafter as CPU), a counter 17 for displaying the resulted data measured in a digital form, a CRT display 18, a key board 19, and a printer for printing out the resultant measurement data.

The CPU 16 included in the main frame 16 collects the measurement data of the workpiece and calculated the inclination and shape of the measured workpiece by processing the measurement data.

Therefore, the inclination of the workpiece can be compensated so that the reference line of the workpiece coincides with the X-axis or the Y-axis by controlling the rotatable table to rotate through a desired angle.

The operation of the optical measuring device of this invention will be described hereunder.

First, a workpiece to be measured is mounted on the rotatable table 1, and secured thereto by predetermined jigs provided on the rotatable table 1.

Then the work is illuminated by a light emitted from the switched ON light source mounted inside of the projection device 7 provided over the rotatable table 1.

At this time, the projecting lens 9 of the projection device 7 is automatically focused on the workpiece to be measured by the zoom function thereof.

The light emitted from the light source 22 and passed through the projection lens 9, is projected onto the projecting screen 20 in an enlarged form.

Then the image of the workpiece projected onto the projecting screen 20 is measured by determining the variations of the amount of light and the inclination to the X-axis or Y-axis, respectively, by the edge sensor 10.

In the actual procedure, since the edge sensor 10 is fixed, the tracing of the contour portion displayed on the projecting screen, can be carried out by moving the movable stage along the X-axis and Y-axis.

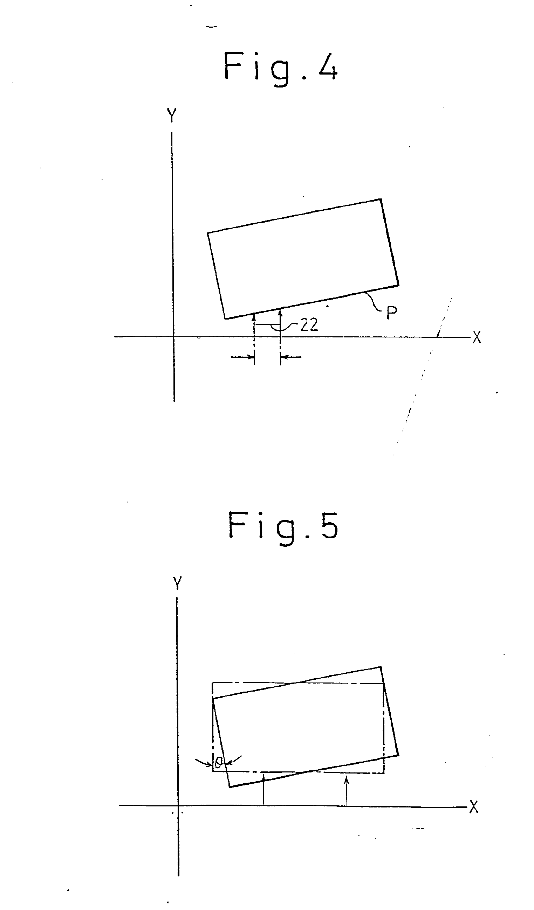

Namely, to explain the operation of this invention while referring to Figure 4, the movement of the movable stage is performed by controlling the rotation of the stepping motors 3 and 4, utilizing a suitable program, to move the focal point over the contour of the workpiece along the line shown by an arrow 22.

For example, assuming a contour P is to be measured, the focal point is first moved to the right along the X-axis by 1 μ, as one operation, and thereafter, is moved upward along the Y-axis to the point at which the edge sensor can discriminate the existence of the contour.

When the edge sensor discriminates the existence of the contour, the data of the X-Y axis coordinate of the contour at that time is registered in the CPU, and successively, the focal point is again moved in the same direction along the X-axis by 1 μ and then is moved again upward along the Y-axis to the point at which the edge sensor can discriminate the existence of the contour, and the measurement of the contour portion can be carried out by repeating the movements described above.

In the example mentioned above, the distance which the focal point is moved at one time may be set in accordance with the accuracy required, and may be within a range of 0.1 μ ∿ 5 mm.

This operation is performed by transmitting data output from the edge sensor to the data processing portion of the CPU, and by rotating the stepping motors 3 and 4 through a very small angle while simultaneously registering the data consisting of X and Y coordinates, respectively, in the CPU, although this operation will be carried out instantly.

When such an operation is carried out from the left end portion of the contour P to the right end portion thereof, the straight line P of the contour is registered, and therefore, the inclination angle θ is calculated in the data processing portion of the CPU by comparing the straight line with the reference line of the X or Y axis coordinate.

Thereafter, the straight line is adjusted and made parallel to, for example, the X-axis by rotating the stepping motor 5 through a desired angle by an output of the resultant data thereto.

In this invention, to coincide the straight line P of the work with the X-axis, the movable stage may be moved to a desired position by rotating the stepping motor 3 and 4 for the X-axis and Y-axis respectively, before or after the operation for making the reference line of the workpiece parallel with the X-axis or Y-axis.

These operations are carried out automatically but may be carried out manually utilizing, for example, a joy-stick 21.

After the operation for making the reference line of the workpiece parallel with the X-axis or Y-axis is completed, the contour of the workpiece outlined by the optical system of the projecting device 7 is detected by the edge sensor 10.

Also, in this invention, the shape of workpiece can be obtained by a data processing method utilizing a suitable program without a further detection of the same by the edge sensor because, as mentioned above, the coordinate data related to the each position on the measured contour P is already registered in the CPU.

While this operation is performed, the amount of light from the light source 22 read by the edge discriminating circuit 24 is always constant, because of the amount of light from the light source 22 is adjusted by the light source sensor 23.

The final measurement data obtained by processing the measurement data of the shape of the work or the like in the controlling and data processing portion 14, is displayed in digital form on the counter 17, and simultaneously displayed on the CRT display 18 and printed out by the printer.

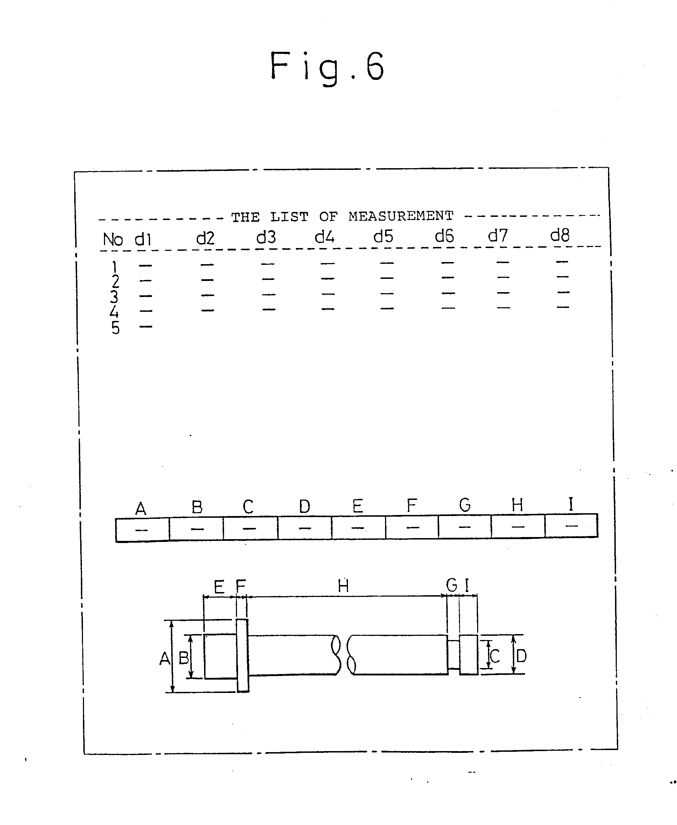

An example of such a printed out form is shown in Figure 6.

Note, the method of this invention, is not restricted to application to the contour of a workpiece having a straight line but may be also applied to the contour of a workpiece having a curved line.

Several applications of the optical measuring device of this invention will be explained hereafter.

Figure 10 shows an example in which a control operation of a die and a product thereof of a pin for a reel is carried out by measuring a diameter of the pin (P-Q) at a position E spaced from a flange portion B by, for example, 80 μm.

In a conventional method, the accuracy of the measurement was not increased because the corner portion R was not clear, but in this invention, the measurement operation can be carried out in such a way that, by first setting a reference line on the flange portion B, the data $Y_c$ and $Y_d$ , which are a distance between the points C and D on the flange portion from the Y-axis respectively, is measured and then the rotatable table is rotated to make both data equal, and thereafter, the diameter (P-Q) is measured.

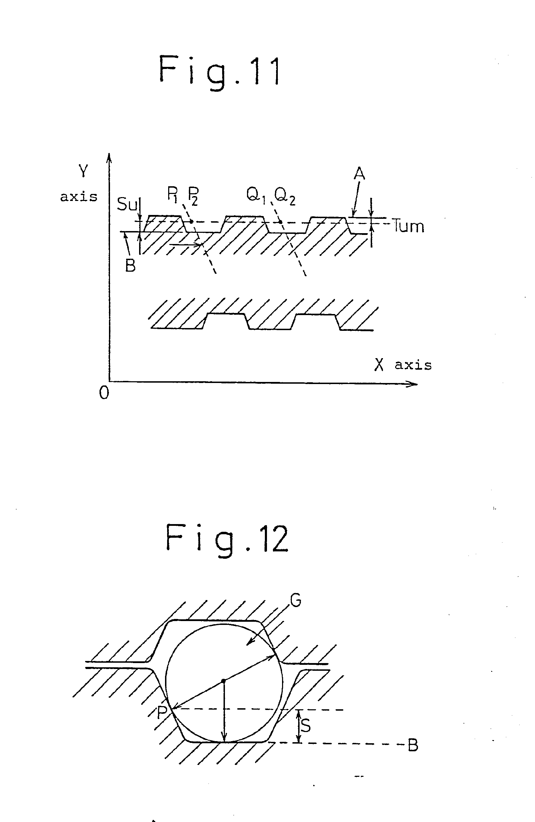

Figures 11 and 12 show a case in which a pitch of a thread of a precision lead screw is measured, together with variation of the angle of rotation of the screw.

In a conventional method for measuring the same, a contactor is attached in a thread groove thereof and the deviation of the contactor $P_1$ , $P_2$ or $Q_1$ , $Q_2$ is measured. But errors occasionally occur, depending upon the shape of the contactor, because there are delicate curves on the polished surface inside the thread groove, and the contact pressure thereof also causes such errors.

Accordingly, in this example, by utilizing a ball bearing G, being a true sphere, the lead pitch thereof can be measured precisely in such a way that the position of the contact point P of the ball bearing with the inside surface of the thread groove is specifically determined in relation to the already parallelize reference line, for example, a reference line B.

In this case, another reference line A as shown in Figure 11 may be used in this example.

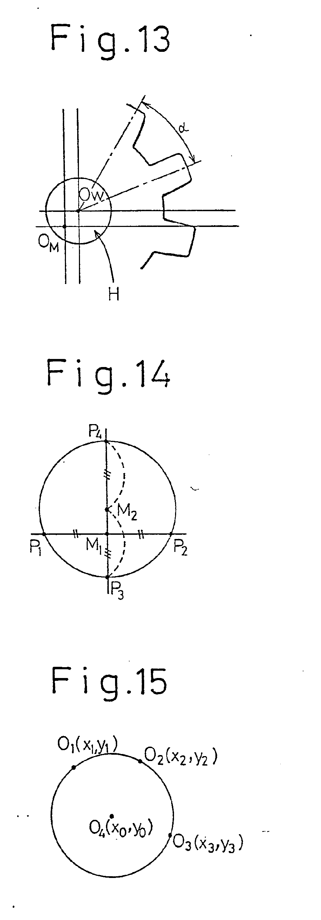

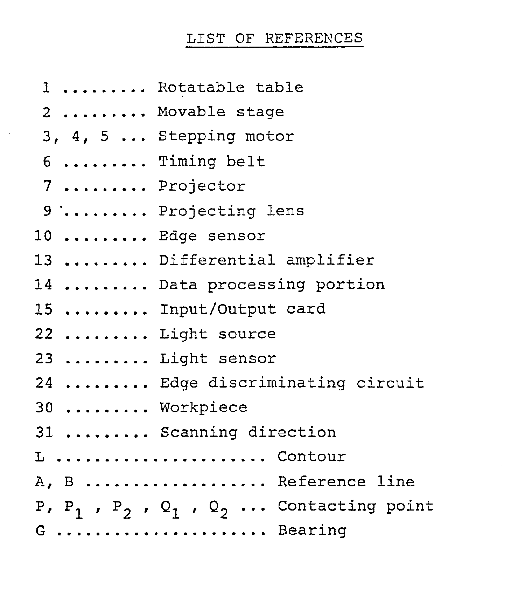

Further, Figures 13 to 15 show a case in which a helix angle $\alpha$ of a gear is measured. (wherein $O_W$ represents a center of the workpiece and $O_M$ represents a rotational center of the measuring machine.)

In a conventional manner, the helix angle is measured by a indexing angle after the gear is mounted on a rotating rod having a diameter matching the shaft hole H of the gear, but it is very difficult to obtain a precise measurement because of an effect caused by a run-out or a vibration of the gear.

But, in the optical measuring device of this invention, an accurate measurement of the helix angle can be obtained by performing an operation for coinciding reference points, as one of the applications of the technology in which a reference line is parallelized.

In this invention, the reference points may be determined by the theory that the center of a circle exists on a median line of the line $P_1$ and $P_2$ as shown in Figure 14, and another theory that a center of the circle covering three points is univocally determined.

Namely, first the coordinate data of the center of the shaft hole H of a work is measured by a method as shown in Figure 14, and the data thus obtained is registered as $O_1$.

Next, another coordinate data of the center thereof is measured in the same manner as described above, after rotating the rotatable table with the workpiece mounted thereon, through a suitable angle, and the data thus obtained is registered as $O_2$, and this measuring method is repeated to obtain separate coordinate data of the center thereof and the data thus obtained is registered as $O_3$.

As mentioned above, the coordinate data of the center of the rotatable table $O_4$ can be determined, the center of the rotatable table and the center of the workpiece can be coincided by adjusting a deviation of the coordinate system between the coordinate data of the center of the rotatable table $O_4$ and the data $O_3$ which is measured in the final step mentioned above.

As explained above, in this invention, remarkably precise measurement data can be obtained because the line at which the edge sensor crosses the contour line can be maintained at a right angle, and moreover, a rapid measuring operation can be performed in this invention.

Furthermore, a three dimensional contour of the workpiece can be measured in this invention, as well as a two dimensional contour thereof, by rotating the workpiece at a right angle in a vertical direction by using suitable jigs on the rotatable table.

Further, in this invention, extremely precise measurement data can be also obtained because errors in the accuracy of the measurement caused by variations of the amount of light from the light source are eliminated by adjusting the amount of light from the light source by the light source sensor, as mentioned above.

As described above, in this invention, the operation for making the reference line of the workpiece parallel with the X-axis or Y-axis can be carried out extremely rapidly and accurately without the need for a manual operation, and the precision thereof will remain unchanged even during a cyclic operation, and further, in this invention, the dimensional data of the workpiece can be simultaneously measured, and accordingly, in this invention, not only an efficient and power-saving measuring operation but also a measuring method in which the same result can be obtained every time, even when such a measuring operation is performed by anybody, can be obtained.

## CLAIMS

1. An optical measuring device which comprises a movable stage, a rotatable table on which a workpiece is mounted and provided on said stage, an optical system for illuminating said workpiece with a light, an edge sensor for reading a contour of said workpiece configurated by said optical system and a data processor which carries out at least one controlling operation selected from among an operation for controlling a movement of said stage corresponding to information from said edge sensor, an operation for controlling a rotational movement of said rotatable table and an operation for displaying measurement data of the workpiece.

2. An optical measuring device according to claim 1, wherein a compensating circuit is used for compensating variations in the amount of light from a light source in said optical system.

0296252

# Fig.1

# Fig.2

# Fig.3

# Fig.4

# Fig.5

# Fig.6

```
---------------- THE LIST OF MEASUREMENT ----------------
No  d1      d2      d3      d4      d5      d6      d7      d8
 1  —       —       —       —       —       —       —       —
 2  —       —       —       —       —       —       —       —
 3  —       —       —       —       —       —       —       —
 4  —       —       —       —       —       —       —       —
 5  —
```

```
    A     B     C     D     E     F     G     H     I
    —     —     —     —     —     —     —     —     —
```

# Fig.7

# Fig.8

# Fig. 9

# Fig. 10

# Fig.11

# Fig.12

# Fig.13

# Fig.14

# Fig.15

0296252

## LIST OF REFERENCES

| | | |
|---|---|---|
| 1 | ........ | Rotatable table |
| 2 | ........ | Movable stage |
| 3, 4, 5 | ... | Stepping motor |
| 6 | ........ | Timing belt |
| 7 | ........ | Projector |
| 9 | ........ | Projecting lens |
| 10 | ........ | Edge sensor |
| 13 | ........ | Differential amplifier |
| 14 | ........ | Data processing portion |
| 15 | ........ | Input/Output card |
| 22 | ........ | Light source |
| 23 | ........ | Light sensor |
| 24 | ........ | Edge discriminating circuit |
| 30 | ........ | Workpiece |
| 31 | ........ | Scanning direction |
| L | ............... | Contour |
| A, B | ............... | Reference line |
| P, $P_1$, $P_2$, $Q_1$, $Q_2$ | ... | Contacting point |
| G | ............... | Bearing |

# INTERNATIONAL SEARCH REPORT

International Application No  PCT/JP88/00012

## I. CLASSIFICATION OF SUBJECT MATTER (if several classification symbols apply, indicate all) [3]

According to International Patent Classification (IPC) or to both National Classification and IPC

Int.Cl[4]  G01B11/24, G01B9/08

## II. FIELDS SEARCHED

### Minimum Documentation Searched [4]

| Classification System | Classification Symbols |
|---|---|
| IPC | G01B11/24, G01B9/08, G01B11/30 |

### Documentation Searched other than Minimum Documentation to the Extent that such Documents are Included in the Fields Searched [5]

| | |
|---|---|
| Jitsuyo Shinan Koho | 1937 – 1981 |
| Kokai Jitsuyo Shinan Koho | 1971 – 1986 |

## III. DOCUMENTS CONSIDERED TO BE RELEVANT [14]

| Category [*] | Citation of Document, [16] with indication, where appropriate, of the relevant passages [17] | Relevant to Claim No. [18] |
|---|---|---|
| X | Morimoto Yoshio "Toeiki" 25 December 1962 (25. 12. 62) P119-120 | 1 |
| Y | Mitsutoyo Giho No. 25, June 1984 Itabashi Tatsuo "Seimitsu Banno Toeiki PJ311" P87-90 | 1 |
| X | JP, A, 58-30608 (Oki Electric Industry Co., Ltd.) 23 February 1983 (23. 02. 83) Fig. 2 (Family: none) | 2 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| March 2, 1988 (02. 03. 88) | March 22, 1988 (22. 03. 88) |
| International Searching Authority [1] | Signature of Authorized Officer [20] |
| Japanese Patent Office | |

Form PCT/ISA/210 (second sheet) (October 1977)